# EUROPEAN PATENT SPECIFICATION

(11) **EP 4 051 294 B1**
(45) Date of publication and mention of the grant of the patent: **13.12.2023**
(21) Application number: 20793183.3
(22) Date of filing: 15.10.2020
(51) Int. Cl.: A61K 33/42, A61K 41/00, A61P 35/00

(54) **USE OF EXFOLIATED BLACK PHOSPHORUS FOR THE TREATMENT OF PROSTATE CANCERS**
VERWENDUNG VON EXFOLIERTEM SCHWARZEM PHOSPHOR ZUR BEHANDLUNG VON PROSTATAKREBSRANKUNGEN
UTILISATION DE PHOSPHORE NOIR EXFOLIÉ POUR LE TRAITEMENT DE CANCERS DE LA PROSTATE

(30) Priority: 29.10.2019 IT 201900019958
(43) Date of publication of application: 07.09.2022
(73) Proprietor: Materias S.r.l., 80122 Napoli (NA) (IT); Raucci, Maria Grazia, 81025 Marcianise (CE) (IT); Ambrosio, Luigi, 80044 Ottaviano (NA) (IT); Fasolino, Ines, 81100 Caserta (IT); Caporali, Maria, 50019 Sesto Fiorentino (FI) (IT); Serrano Ruiz, Manuel, 50019 Sesto Fiorentino (FI) (IT); Peruzzini, Maurizio, 50018 Scandicci (FI) (IT)
(72) Inventor: RAUCCI, Maria Grazia, 81025 Marcianise (CE) (IT); AMBROSIO, Luigi, 80044 Ottaviano (NA) (IT); FASOLINO, Ines, 81100 Caserta (IT); CAPORALI, Maria, 50019 Sesto Fiorentino (FI) (IT); SERRANO RUIZ, Manuel, 50019 Sesto Fiorentino (FI) (IT); PERUZZINI, Maurizio, 50018 Scandicci (FI) (IT)
(74) Representative: PGA S.p.A.
(86) International application number: PCT/IB2020/059686
(87) International publication number: WO 2021/084361

(56) References cited:
- WO-A1-2020/049475
- CN-A- 109 432 437
- MARIA GRAZIA RAUCCI ET AL: "Exfoliated Black Phosphorus Promotes in Vitro Bone Regeneration and Suppresses Osteosarcoma Progression through Cancer-Related Inflammation Inhibition", ACS APPLIED MATERIALS & INTERFACES, vol. 11, no. 9, 13 February 2019 (2019-02-13), pages 9333-9342, XP055710467, US ISSN: 1944-8244, DOI: 10.1021/acsami.8b21592 cited in the application
- HUI WANG ET AL: "Ultrathin Black Phosphorus Nanosheets for Efficient Singlet Oxygen Generation", JOURNAL OF THE AMERICAN CHEMICAL SOCIETY, vol. 137, no. 35, 26 August 2015 (2015-08-26), pages 11376-11382, XP055710463, US ISSN: 0002-7863, DOI: 10.1021/jacs.5b06025 cited in the application
- JUNDONG SHAO ET AL: "Biodegradable black phosphorus-based nanospheres for in vivo photothermal cancer therapy", NATURE COMMUNICATIONS, vol. 7, no. 1, 30 September 2016 (2016-09-30), XP055710496, DOI: 10.1038/ncomms12967 cited in the application
- XIAOXIAO GE ET AL: "Recent Advances on Black Phosphorus for Biomedicine and Biosensing", ADVANCED FUNCTIONAL MATERIALS, vol. 29, no. 29, 10 May 2019 (2019-05-10), page 1900318, XP055710555, DE ISSN: 1616-301X, DOI: 10.1002/adfm.201900318 cited in the application
- LI GAO ET AL: "Zinc Ion-Stabilized Aptamer-Targeted Black Phosphorus Nanosheets for Enhanced Photothermal/Chemotherapy Against Prostate Cancer", FRONTIERS IN BIOENGINEERING AND BIOTECHNOLOGY, vol. 8, 31 August 2020 (2020-08-31), XP055756771, DOI: 10.3389/fbioe.2020.00769

## Description

### FIELD OF THE INVENTION

The present invention relates to exfoliated black phosphorus for use in the treatment of a prostate cancer with or without irradiation by photothermal or photodynamic therapy.

### BACKGROUND OF THE INVENTION

Nowadays prostate cancer is the most common tumor in worldwide male population. Recent high incidence rates of prostate adenocarcinoma are responsible of 20% of cancer-related deaths in the male Western population. Numerous studies demonstrated that 90% of cases of prostate adenocarcinoma are organ-confined and it is possible to apply a local radiotherapy or prostatectomy. Furthermore, because of its hormone-responsivity the chemical androgen deprivation represents another therapeutic approach. However, the patients often become resistant to the hormone-therapy that shows a transient effectiveness (18-36 months). Another current strategy to treat prostate cancer is the use of AR competitive antagonists alone or in combination with anti-metastatic drugs or immunotherapy. This drug combination is useful for the treatment of not organ-confined prostate adenocarcinoma that often metastasizes in the bones.

Presently, brachytherapy is the most effective radiotherapy method for the local treatment of unresectable localized prostate carcinoma. Brachytherapy can be distinct in low (LDR) and high dose rate (HDR) brachytherapy. Brachytherapy belongs to radiation therapy strategies that are recommended for the curative treatment of male patients with prostate cancer. It consists on the transient or permanent implantation of radioactive sources into or very near target tissues. Indeed, "brachy" from the Greek term "brakhus" means "short" which refers to radiation therapy where radioactive sources are delivered really closed to cancer tissue. Low dose rate brachytherapy is commonly obtained by placing permanently radioactive sources. This method differs from high dose rate (HDR) brachytherapy, where stronger radioactive sources are placed temporarily into the prostate and removed after the delivery of the effective dose.

From a medical standpoint, LDR brachytherapy represents a minimal-invasive procedure for the treatment of prostate cancer due to an accurate implantation of radioactive sources in a specific anatomical location. In addition, from a radiobiological point of view, the controlled dose escalation provided by LDR brachytherapy results more effective in killing tumor cells compared to conventional radiotherapy thus reducing the toxicity risks related to external beam radiation therapy (EBRT) that affect the bladder and rectum. Hence, LDR allows increasing EBTR dose about two times thus improving radiation effectiveness with a lower toxicity.

However, LDR causes some irreversible side effects but less troublesome compared to external-beam radiotherapy due to the radiation sources implantation. Some side effects arise after several weeks and may last for longer. These collateral symptoms include erection problems, inhibition of ejaculation, infertility, bowel problems and difficulty in urine passing with pain.

In the field of regenerative medicine, much effort has been devoted to novel smart biomaterials with photo-thermal and photodynamic properties useful for cancer therapy that can substitute the conventional local radiotherapy. In recent years, numerous studies support the use of Photodynamic Therapy (PDT) as minimally invasive therapeutic approach with a selective cytotoxic activity toward cancer cells.

On this respect, a recently discovered 2D material, named phosphorene, being the P-counterpart of graphene and obtained by exfoliation of crystals of the black allotrope of the element, has attracted the attention of scientists working in the biomedical field intrigued by its very low (or absent) toxicity towards various cells [N. M. Latiff et al., Chem. Eur. J. 2015, 21, 13991-13995]. Being phosphorene the monolayer, the 2D nanomaterial usually employed in the chemical, physical and biomedical applications, is the exfoliated black phosphorus, also named 2D black phosphorus or "2D bP". To date exfoliated black phosphorus, as a novel two-dimensional nano-material, has attracted great attention owing to its anticancer properties in cancer phototherapy, namely photothermal therapy (PTT) or photodynamic therapy (PDT). Recent studies have shown the effectiveness of 2D bP as photodynamic therapy agent for breast cancer (MCF7 breast cancer cells) and melanoma (B16 melanoma cells) treatment [J. Shao et al., Nat. Commun. 2016, 7, 12967]. This activity has been ascribed to the capability of 2D bP to generate singlet oxygen and to act as photosensitizer that, in presence of reactive oxygen species (ROS) and infrared light irradiation, constitutes an essential component of PDT therapy. Notably it is reported that after a near-infrared light irradiation of ultrathin bP nanosheets, singlet oxygen is generated that serves as effective photodynamic therapy (PDT) in cancer treatment [W. Tao et al., Adv Mater. 2017, 29, 1603276].

A previous study on a SAOS-2 osteosarcoma cells in vitro model demonstrated that 2D bP nanosheets possess intrinsic anticancer and anti-inflammatory properties that are independent of near infrared light irradiation [Raucci MG et al., ACS Appl Mater Interfaces. 2019 Mar 6;11(9):9333-9342].

A previous study on a MDA-MB-231 breast cancer cells in vitro and in vivo demonstrated that ultrathin 2D exfoliated black phosphorus nanosheets are effective photosensitizers for the generation of singlet oxygen, rendering their application attractive in breast cancer therapy [Wang H et al., Journal of the American Chemical Society, 2015 August 26; 137(35): 11376-11382].

A review study reported that black phosphorous nanostructures under 808 nm laser irradiation are able to kill nearly all C6 glioma cells as well as MCF7 breast cancer cells; moreover, the review reported that mice treated with both black phosphorous nanosheets and laser irradiation showed decrease of the volume of the tumor in MDA-MB-231 breast tumor-beared Balb/c nude mice [Xiaoxiao G et al., Advanced Functional Materials, 2019 May 10; 29(29): 1900318].

All the studies reported in the prior art cited above are specifically directed at the treatment of a specific tumor line; moreover, none of those documents describes or suggests the use of 2D exfoliated black phosphorus in the treatment of prostate cancer.

### SUMMARY OF THE INVENTION

The Applicant has surprisingly found that 2D exfoliated black phosphorus has effect on prostate cancer with or without irradiation by photothermal or photodynamic therapy.

The Applicant has found that the presence of 2D exfoliated black phosphorus with or without irradiation decreases cell viability and proliferation of prostate adenocarcinoma cells without exerting any cytotoxic effect on prostate healthy cells.

Thus, the Applicant has found that 2D exfoliated black phosphorus exerts anti-proliferative effects on prostate cancer cells without adversely affect normal prostate tissue.

Further, the Applicant has found that the presence of 2D exfoliated black phosphorus with or without irradiation was able to induce a significant increase in ROS generation thus suggesting 2D bP ability of causing oxidative stress and determining a permanent damage in cancer prostatic cells.

The Applicant also observed that in cancer prostatic cells 2D bP without irradiation maintained high nitrites levels induced by LPS and that irradiated 2D bP was able to increase basal levels of nitrite even without LPS stimulation. On the contrary, 2D bP with and without NIR-irradiation was able to decrease nitrite production in healthy prostate cells. Finally, the Applicant has found that in cancer prostatic cells 2D bP induced the caspase-1 expression and determined an increase of pro-inflammatory cytokines, while in healthy prostate cells 2D bP did not induce caspase-1 expression and significantly decrease TGF-β levels.

In other words, the Applicant has surprisingly found that 2D exfoliated black phosphorus (i) induces anti-proliferative and apoptotic effects, with or without any phototherapy treatment, by increasing the production of ROS on cancer prostatic cells, and at the same time (ii) shows an opposite behavior in the case of healthy prostate cells, thus making 2D exfoliated black phosphorus a powerful material in prostate cancer therapy.

Then, the Applicant's results suggest the use of 2D exfoliated black phosphorus in the treatment of prostate cancer as anti-cancer agent with protective properties versus healthy prostate cells.

Accordingly, a first object of the present invention relates to 2D exfoliated black phosphorus for use in the treatment of prostate cancer with or without phototherapy.

### BRIEF DESCRIPTION OF THE FIGURES

Figure 1 shows a SEM image of bulk black phosphorus.
Figure 2 shows a bright field TEM image of exfoliated black phosphorus with scale bar of 1000 nm.
Figure 3 shows the effect of 2D bP on PNT-2 cell proliferation as described in Example 2.1.
Figure 4 shows the effect of 2D bP on PC-3 cell proliferation as described in Example 2.1.
Figure 5 shows the effect of 2D bP on PNT-2 and PC-3 morphological features obtained through immunofluorescence assay as described in Example 2.2.
Figure 6 shows the results of ROS production from PNT-2 (A-C) and PC-3 (B-D) cells with and without infrared stimulation as described in Example 2.3.
Figure 7 shows nitrites production from PNT-2 (A) and PC-3 (B) cells with and without infrared stimulation as described in Example 2.4.
Figure 8 shows optical analysis of PNT-2 and PC-3 morphology after 48 hours of exposure to 2D bP as described in Example 2.4.
Figure 9 shows the effect of 2D bP on intracellular iron amount in PNT-2 and PC-3 cells as described in Example 2.5.
Figure 10 shows the effect of 2D bP on caspases-1 expression in PNT- 2 and PC-3 cells as described in Example 2.6.1.
Figure 11 shows the effect of 2D bP on PNT-2 qualitative caspases-1 expression obtained through immunofluorescence assay as described in Example 2.6.1.
Figure 12 shows the effect of 2D bP on PC-3 qualitative caspases-1 expression obtained through immunofluorescence assay as described in Example 2.6.1.
Figure 13 shows the effect of 2D bP on TGF-β levels in PNT-2 (A) and PC-3 cells (B) as described in Example 2.6.2.
Figure 14 shows the effect of 2D bP on nitrite, IL-10, IL-6 and COX-2 levels in PNT-2 as described in Examples 2.7.1-3.
Figure 15 shows the effect of 2D bP on SOD activity in PNT-2 as described in Examples 2.7.4.

### DETAILED DESCRIPTION OF THE INVENTION

According to the first object, the present invention relates to 2D exfoliated black phosphorus for use in the treatment, preferably in the localized treatment, of prostate cancer with or without phototherapy.

2D exfoliated black phosphorus is able to inhibit the proliferation and viability of prostate cancer cells and at the same time is able to protect the viability of healthy prostate cells.

Prostate cancer cells include primary prostate cancer cells and advanced prostate cancer cells. Primary prostate cancer cells are organ confined. Advanced prostate cancer refers to high histological grade, organ confined prostate cancer cells.

For the purpose of the present description and the appended claims, the term "localized treatment" means that the 2D exfoliated black phosphorus is used to treat organ confined prostate cancer cells.

According to an embodiment of the present invention the 2D exfoliated black phosphorus is locally administered within the prostate tumor mass.

Local administration can be performed by method known in the art, such as, for example by dispersing 2D exfoliated black phosphorus in a physiological injectable solution.

In a preferred embodiment, 2D exfoliated black phosphorus can be used as such or incorporated into a proper carrier, such as polymeric microspheres, able to release the 2D exfoliated black phosphorus within the prostate tumor mass. According to an embodiment of the present invention, 2D exfoliated black phosphorus is used to treat prostate cancer with phototherapy. Phototherapy can be distinguished in photothermal therapy (PTT) or photodynamic therapy (PDT). Photodynamic therapy (PDT), is a form of phototherapy involving light and a photosensitizing chemical substance, used in conjunction with molecular oxygen to elicit cell death (phototoxicity).

Photothermal therapy (PTT) is a minimally invasive, local treatment modality that mainly relies on an optical absorbing agent, also known as photosensitizer, which can absorb energy and convert it into heat upon stimulating by an electromagnetic radiation such as radiofrequency, microwaves, near infrared irradiation, or visible light.

2D exfoliated black phosphorus can be obtained by liquid exfoliation of bulk crystalline black phosphorus under the action of ultrasounds following the procedure disclosed by M. Serrano-Ruiz et al., Adv. Mater. Interfaces 2016, 3, 1500441. Bulk crystalline black phosphorus can be prepared from red phosphorus according to the procedure disclosed by T. Nilges et al., J. Solid State Chem. 2008, 181, 1707-1711.

Generally, bulk crystals of black phosphorus can be suspended in polar aprotic solvent, such as dry dimethylsulfoxide (DMSO), and then mixed with deoxygenated water in order to get a molar ratio P/H₂O ranging from 14:1.5 to 3:1, more preferably of about 2:1.

Preferably, the mixture is poured in a vial and deoxygenated under vacuum and nitrogen. The vial is sealed and sonicated (37 kHz) for at least 2 days, preferably from 3 to 6 days, at temperature ranging from 20 to 40°C, preferably ranging from 25° to 35°C, more preferably about 30°C.

Afterwards, the vial is opened, added with degassed polar solvent, such as ethanol, and the suspension is centrifuged for at least 10 minutes, preferably from 20 to 60 minutes, more preferably about 30 minutes at 5,000-15,0000 rpm, preferably 7,000-12,000 rpm, more preferably about 9500 rpm.

The surnatant is removed, fresh polar aprotic solvent such as acetone is added, and the suspension is again centrifuged for at least 10 minutes, preferably from 20 to 60 minutes, more preferably about 30 minutes at 5,000-15,0000 rpm, preferably 7,000-12,000 rpm, more preferably about 9500 rpm.

The resulting dark grey powder is re-suspended by sonication (for 0.5-5 minutes, preferably 0.5-2 minutes, more preferably approximately one minute) in a polar protic solvent, such as isopropanol or methanol and the desired amount of suspension is drop-casted on a well plate and left slowly to evaporate the solvent under a stream of inert gas, such as nitrogen, in the dark to get a dry film of exfoliated black phosphorus on the surface of the well plate.

The invention will be further described in the following examples, which do not limit the scope of the invention described in the claims.

### EXAMPLE 1

### 2D black phosphorus preparation

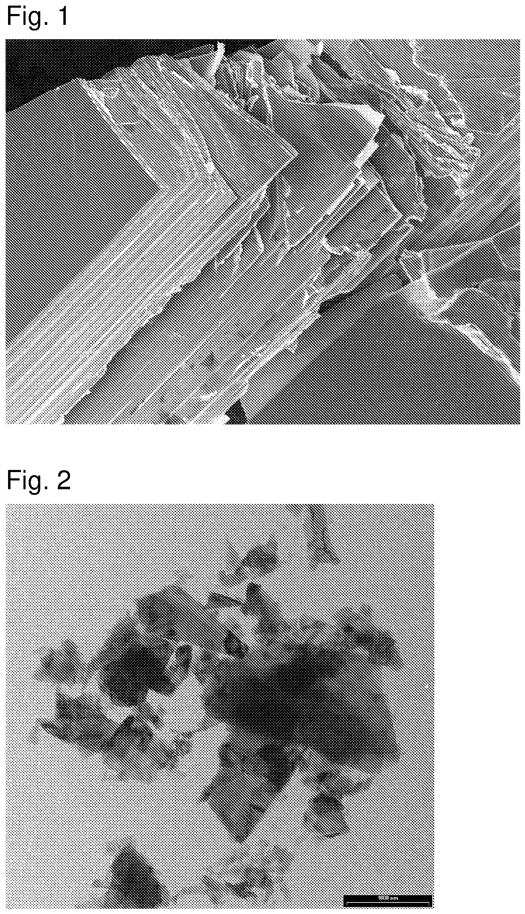

Bulk crystalline black phosphorus was prepared from red phosphorus according to the procedure disclosed by T. Nilges et al., J. Solid State Chem. 2008, 181, 1707-1711 and was characterized by scanning electron microscopy (Fig. 1), energy dispersive X-ray spectroscopy (EDX), powder X-ray diffraction and Raman spectroscopy.

Red phosphorus (99.999% purity) was purchased from Aldrich and used as received.

The SEM images were obtained from a FEI Quanta 200 FEG field-emission scanning electron microscopy with an accelerating voltage of 30 kV and a working distance of 9.1 mm.

The bulk material was subjected to liquid exfoliation under the action of ultrasounds following the procedure disclosed by M. Serrano-Ruiz et al., Adv. Mater. Interfaces 2016, 3, 1500441.

Crystals of black phosphorus (5.0 mg, 0.161 mmol) were suspended in 5.0 mL of dry DMSO and then deoxygenated water (1.0 µL, 0.056 mmol) was added in order to get a molar ratio P/H₂O = 1-0.6. The mixture was deoxygenated under vacuum and nitrogen, the vial was sealed and sonicated (37 kHz) for 5 days at 30°C. Afterwards, the vial was opened, added with degassed ethanol (10.0 mL), and the suspension was centrifuged for 30 minutes at 9500 rpm. The surnatant was removed, degassed acetone (10.0 mL) was added, and again the suspension was centrifuged at 9500 rpm for 30 min. The resulting dark grey powder was re-suspended by sonication (approximately one minute) in isopropanol (10.0 mL) and the desired amount of suspension was drop-casted on the well plate and left slowly to evaporate the solvent under a stream of nitrogen in the dark.

Exfoliated 2D black phosphorus was characterized using transmission electron microscopy (Fig. 2), energy dispersive X-ray spectroscopy (EDX), atomic force microscopy (AFM), powder X-ray diffraction (PXRD), Raman spectroscopy and X-ray photoelectron spectroscopy (XPS). Powder X-ray diffraction of the dark gray solid which settled down after centrifugation was also carried out to confirm the nature of the sample and its purity. The XRD results demonstrated the presence of only few peaks, which correspond to the 020, 040, and 060 reflections of BP, while any other peaks were absent or extremely weak.

The TEM images were obtained by using a Philips instrument operating at an accelerating voltage of 100 kV.

Raman spectra were collected with a custom-built microspectrometer. The 488 nm line from an Ar laser was focused on the sample with a 63× objective with a numerical aperture of 0.9. The power on the sample was 0.3 mW. Light emitted by the sample was dispersed by an HR550 Jobin-Yvon monochromator and the spectrum collected by a Synapse CCD.

EDX (SEM and TEM) : Bruker Quantax 800 EDX having an energy resolution of ≈130 eV, and operated with an electron beam energy of 5 kV.

X-ray powder diffraction (XRD) data were collected with an X'Pert PRO diffractometer with Cu Ko radiation (A = 1.5418 Å). XRD spectra were acquired at room temperature with a PANalytical X'PERT PRO diffractometer, employing Cu Ko radiation (λ = 1.54187 Å) and a parabolic MPD-mirror for Cu radiation.

### EXAMPLE 2

### BIOLOGICAL INVESTIGATION

Biological investigations were performed using PNT-2 (human Normal prostate epithelium immortalized with SV40) and PC-3 (human Caucasian prostate adenocarcinoma) cell lines purchased from Sigma-Aldrich (Milano, Italy). Cells were cultured in 75 cm² cell culture flask in RPMI 1640 and Dulbecco's Modified Eagle's Medium (DMEM) respectively supplemented with 10% Fetal Bovine Serum (FBS), antibiotic solution (streptomycin 100 µg/ml and penicillin 100U/ml, Sigma Chem. Co) and 2 mM L-glutamine. PNT-2 cells at passage 9-11 and PC-3 cells at passage 11-15 were used for all the experimental tests and were grown at 37°C in an incubator containing humidified atmosphere with 5% CO₂ and 95% air.

### Instruments and materials

Immuno-fluorescence analysis was carried out by Leica TCS sp8 confocal microscope.

Enzyme-linked immunosorbent assay (ELISA) was performed by using a commercial ELISA kit provided by Affimetrix Italia s.r.l.

Optical density was measured with a UV-VIS spectrophotometer Victor X3 Multilabel Plate Reader, Perkin Elmer. Fluorescence measurements were performed using a microplate reader, Victor X3, Perkin Elmer, with the excitation wavelength of 485 nm and the emission wavelength of 538 nm.

Optical images were acquired using Motic AE31 Inverted Biological Microscope.

Alamar blue assay was purchased from AbD Serotec, Milan, Italy.

### Example 2.1 - Cell proliferation

Cell proliferation was analyzed on PNT-2 and PC-3 cells in presence of 2D bP (5-75 µg/mL) with and without NIR-stimulation. The range of 2D bP concentrations 5-75 µg/mL was chosen in order to find the best concentration useful to kill cancer cells but not toxic for the surrounding healthy prostate cells.

PNT-2 and PC-3 cells were seeded onto 2D bP at density of 10⁴ and cell behavior in terms of cell proliferation and ROS production was analyzed after two NIR-irradiation cycles of 15 minutes each. In particular, after 24 hours of cell-material interaction, the cells were irradiated for 15 minutes as first time and then incubated overnight at 37°C. Later, the cells were irradiated for a second time and cell proliferation was tested by Alamar Blue assay. Cells in plate without coating were used as control. After 24 and 72 hours of seeding onto 2D bP (5-75 µg/mL), with and without NIR-irradiation, Alamar Blue^{™} reagent was incubated in each well. Optical density was measured with a UV-VIS spectrophotometer (Victor X3 Multilabel Plate Reader, Perkin Elmer) at wavelengths of 570 and 600 nm after 4 hours of incubation at 37°C that is time necessary for the cells to convert resazurin to resorufin. The optical density detected is directly proportional to metabolic activity of live cells.

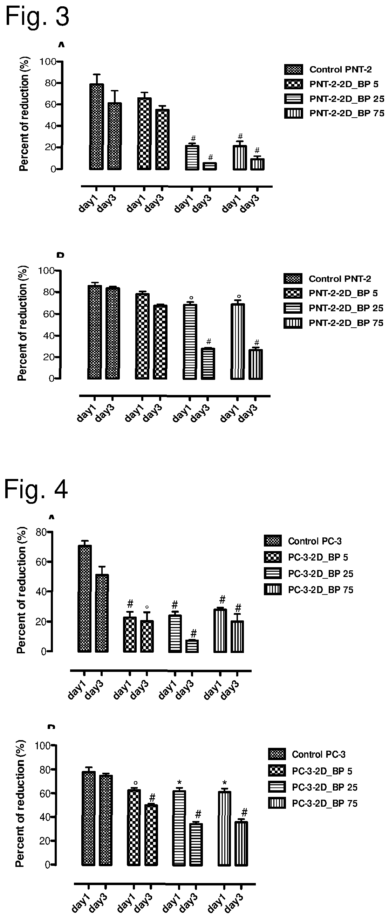

Figure 3 and Figure 4 illustrate the effect of 2D bP on PNT-2 and PC-3 proliferation, respectively.

The experiments performed on PNT-2 and PC-3 cell proliferation using Alamar Blue assay have shown that irradiated and not irradiated 2D bP 5 µg/mL did not exert cytotoxic effects on PNT-2 after 1 and 3 days of exposure. The same result was obtained in presence of not irradiated 2D bP 25 µg/mL at day 1 of cell culture. Conversely, not irradiated 2D bP 75 µg/mL at day 3 and irradiated 2D bP 25-75 µg/mL at all time points significantly (°p≤0.001; ^{#}p≤50.0001) decreased cell viability of healthy prostate cells (PNT-2) compared to control. Meanwhile, the irradiated and not irradiated lowest concentration (5 µg/mL) of 2D bP induced a significant (°p≤0.001; ^{#}p≤0.0001) decrease in PC-3 (cancer cells) proliferation after 1 and 3 days of exposure compared to control. However, this effect results more significant in presence of NIR-irradiation. The best inhibition in PC-3 proliferation was obtained after 3 days of irradiated 2D bP exposure at concentration of 75 µg/mL (^{#}p≤0.0001) but this concentration is toxic for PNT-2 cells. For this reason, the majority of analyses were performed using the lowest concentration of 2D bP (5 µg/mL) that decreased PC-3 proliferation without exerting any cytotoxic effect on PNT-2 healthy cells.

### Example 2.2 - Cell morphology

Furthermore, in order to study morphological changes, immunofluorescence analysis was also performed. Immunofluorescence analysis was carried out using a confocal laser scanning microscopy (Leica TCS sp8 confocal microscope). For this purpose, the cells cultured in 8-well culture chamber at a density of 2×10⁴cells/slides. After 48 hours of exposure to 2D bP, in presence and in absence of NIR-irradiation, cells were fixed in 4% formaldehyde at room temperature for 10-30 minutes, washed thrice with PBS and permeabilized with 0.1% Triton X-100 in PBS for 1 hour. Cells without 2D bP treatment were used as plate controls. Then, the cells were washed 2-3 times in PBS and phalloidin-FITC conjugated working solution was incubated for 1 hour. After that, samples stained with phalloidin were washed 3 times with PBS and cell images were acquired with a confocal microscope.

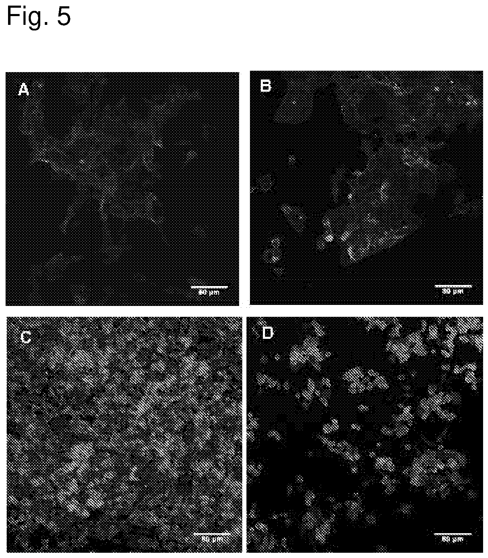

Morphological examinations obtained through immunofluorescence assay are shown in Figure 5.

In Figure 5, PNT-2 after 24 hours of 2D bP (B) exposure showed the same morphology and density of the control (A). Conversely, PC-3 cells treated with 2D bP for 24 hours have lost their specific tapered shape (D) thus indicating cell-suffering condition compared to control where cells appeared well spread and at higher density (C).

### Example 2.3 - ROS production

The increase of Reactive oxygen species (ROS) often induces cell death through the activation of apoptosis mechanisms such as caspases induction. Recently, selective ROS induction in cancer cells has been paid much attention as strategy to inhibit cancer cell proliferation. It is well known that cancer cells spontaneously produce high levels of ROS deriving of an increased metabolic activity and a constant crosstalk with infiltrating immune cells. Hence, it was established that the increase of cancer cells immune response in terms of growth factors and cytokines stimulation increases ROS generation. 2D bP capability to increase ROS production in PC-3 prostate cancer cells compared to healthy PNT-2 cells in presence and in absence of NIR-irradiation was investigated.

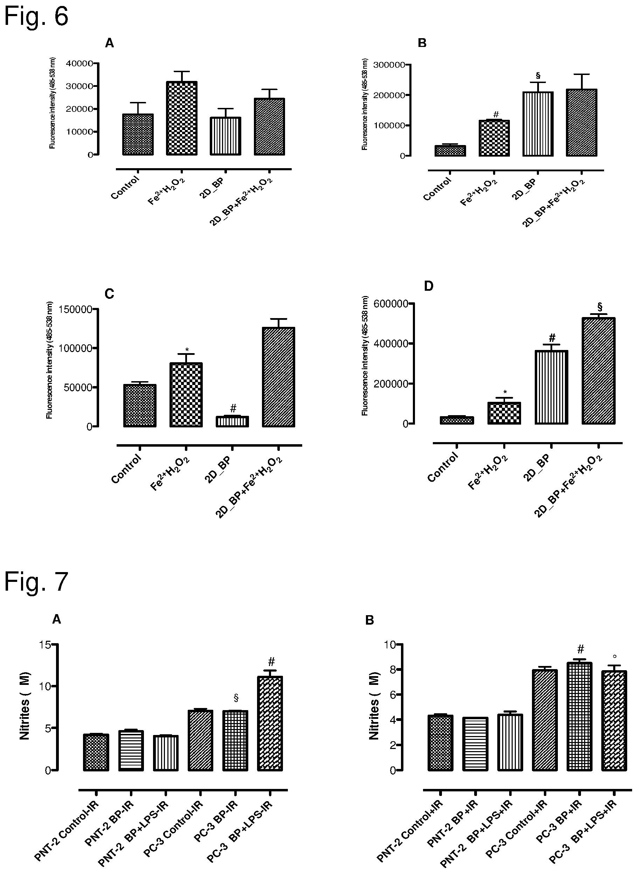

ROS species production in cells seeded onto 2D bP (5 µg/mL) was monitored by using the fluorescent probe DCFH-DA that, in presence of ROS, is rapidly oxidized to highly fluorescent dichlorofluorescein (DCF). The fluorescence intensity detected is directly proportional to the amount of ROS produced intracellularly. In order to measure ROS production, cells were incubated for 1 hour with 100 µM solution of DCFH-DA in Hanks' balanced salt solution containing 1% FBS; after the incubation, cells were washed thrice with PBS and treated with the Fenton's reagent (H₂O₂/Fe²+ 2 mM) for 3 hours at 37°C. The DCF fluorescence intensity was detected through a fluorescent microplate reader (Victor X3, PerkinElmer), with the excitation wavelength of 485 nm and the emission wavelength of 538 nm. Figure 6 illustrates the results of ROS production from PNT-2 (A-C) and PC-3 (B-D) cells with and without infrared stimulation.

The results suggested that the exposure of PNT-2 (healthy cells) to H₂O₂/Fe²+ at concentration of 2 mM (oxidative agent) induced an increase in ROS production (Fig. 6A). Irradiated 2D bP (5 µg/mL) did not significantly change basal ROS levels compared to control (Fig. 6A). However, a pre-treatment with 2D bP (2D bP H₂O₂/Fe²+) for 24 hours reduced ROS generation induced by H₂O₂/Fe²+ (2 mM) compared to H₂O₂/Fe²+ (2 mM) alone (Fig. 6A). However, in the case of PC-3 cells, stimulation with H₂O₂/Fe²+ (2 mM) induced a significant (^{#}p≤0.0001) increase in ROS production. Furthermore, irradiated 2D bP with and without H₂O₂/Fe²+ (2 mM) stimulation was able to induce a significant increase in ROS generation thus determining a permanent damage in cancer cells (Fig. 6B). The exposure of PNT-2 and PC-3 cells (healthy and cancer cells) to H₂O₂/Fe²+ (2 mM) significantly (*p≤0.05) increases ROS production compared to control. Not irradiated 2D bP significantly (#p≤0.0001) reduced basal ROS levels compared to control on PNT-2 cell line (Fig. 6C). Conversely, not irradiated 2D bP did not inhibit ROS generation induced by H₂O₂/Fe²+ (2 mM) in PNT-2 cells compared to H₂O₂/Fe²+ (2 mM) alone (Fig. 6C). In the case of cancer cells (PC-3) not irradiated 2D bP with and without H₂O₂/Fe²+ (2 mM) stimulation induced a significant (^{#'§p}≤0.0001) increase in ROS production thus suggesting 2D bP ability of causing oxidative stress in cancer prostatic cell lines (Fig. 6D).

### Example 2.4 - Effect of 2D exfoliated black phosphorus on nitrites

Several previous studies have demonstrated an increasing of hydrogen peroxide and nitric oxide levels in tumour cells in response to inflammatory stimuli such interferon γ (IFNy), TNFo and Lipopolysaccharide thus confirming a higher crosstalk with immune system. Additionally, many recent findings have shown that immune response play a pivotal role in cancer progression and ROS induced by neutrophils and macrophages contribute to kill tumour cells. It was also established that during inflammatory response immune cells such as macrophages also generate nitric oxide which combined to superoxide generates to peroxinitrite radicals that contribute to induce tumour cell apoptosis. Hence, the biological response also in terms of nitric oxide production was evaluated by simulating an inflammatory condition obtained by stimulating PNT-2 and PC-3 with LPS in presence of irradiated and not irradiated 2D bP for 24 hours.

Stable metabolites of nitric oxide were detected using Griess assay. For Griess assay, cells were plated in a 48-multiwell plate at density of 5×10⁴ cells and were exposed to 2D bP (5 µg/mL) for 24 hrs. Later, cells were treated with Lipopolysaccharide (flogistic agent) at concentration of 1µg/mL as positive control for 24 and 72 hours. After 2D bP exposure with and without NIR-irradiation cycles, 100 µL of cell supernatant was transferred into a 96-well plate and incubated with an equal volume of Griess reagent. After 1 hour of incubation at RT, the absorbance was measured at a wavelength of 550 nm.

The results illustrated in Figure 7 demonstrated that 2D bP with and without NIR-irradiation was able to decrease nitrite production in healthy PNT-2 cells (7A). An opposite behaviour was observed in PC-3 cancer cells, where 2D bP without irradiation maintained high nitrite levels induced by LPS (7A). This effect is more significant in presence of NIR-irradiation as showed in Figure 7B. Indeed, irradiated 2D bP was able to increase also basal levels of nitrite without LPS stimulation. The effect of 2D bP on nitric oxide generation confirmed the potential anticancer activity related to a possible saturation of radical species responsible of apoptosis induction in cancer tissues.

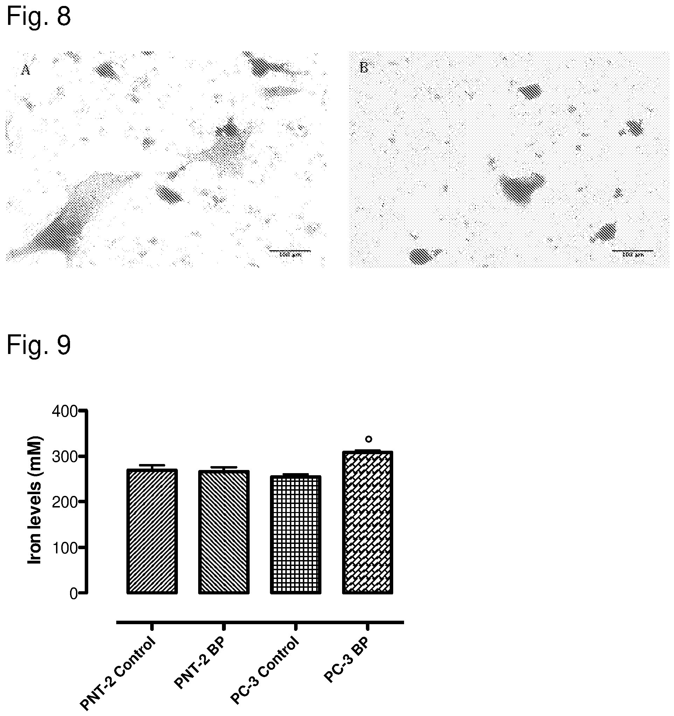

To deepen the effect of oxidative stress on PNT-2 and PC-3 morphology, after cell-material interaction for 48 hours, qualitative analysis was performed using an optical microscope (Motic AE31 Inverted Biological Microscope) after cell fixation in 4% formaldehyde overnight. Figure 8A is representative of PNT-2 after the interaction of 48 hours with 2D bP and a good cell spreading was observed. Conversely, PC-3 cells showed apoptotic cell shape after 48 hours of interaction with 2D bP without NIR-irradiation (Figure 8B).

### Example 2.5 - Effect of 2D exfoliated black phosphorus on iron levels

In order to discover the pathway involved in 2D bP mechanism of action, the intracellular iron amount was measured on the basis of previous studies that have demonstrated that iron overload determined an excess of radical species with the saturation of the buffer mechanisms thus inducing a dramatic increasing of oxidative stress. Intracellular iron amount was measured using Red Prussiate reagent to obtain a colorimetric iron dosing reaction. A standard curve was built using different concentrations of Iron nitrate and a blank solution was prepared with 100 µL of distilled water, 100 µL of 5 N HCI and 100 µL of Red Prussiate reagent. For iron detection PNT-2 and PC-3 (5×10⁵/well) were seeded onto 2D bP with and without NIR-irradiation. Then, cells were trypsinized and incubated with 5N HCl solution at 37°C for 3 days. After this time 100 µL of samples was collected and incubated with 100 µL of Red Prussiate reagent for 15 minutes. The optical density (OD) was recorded at 450 nm using a spectrophotometer (Victor X3 Multilabel Plate Reader, Perkin Elmer).

The induction of cytotoxicity in PC-3 was confirmed by the increase of iron levels (Figure 9) and the oxidative stress after cell-material interaction. In addition, 2D bP did not change basal values of iron in PNT-2 thus suggesting the ability of 2D bP to protect buffer mechanisms (antioxidant activity) in physiological conditions.

### Example 2.6 - Effect of 2D exfoliated black phosphorus in apoptosis mechanism induction: caspase-1 expression and TGF-β secretion

### Example 2.6.1 - Caspase-1 expression

To investigate apoptosis induction by 2D bP, caspase-1 expression apoptotic enzyme was evaluated in absence of NIR-irradiation with western blot analysis and immunofluorescence analysis.

Protein extracts were collected from PNT-2 or PC-3 cells seeded at density of 5×10⁵ onto 2D bP. The protein amount was determined using Bradford method and the proteins were subjected to electrophoresis on 10-15% polyacrylamide precast gel. Later, the proteins were electrophoretically transferred to a nitrocellulose membrane and blocked in 5% non-fat dry milk buffer for 1 hour. After blocking, the membranes were incubated with Caspase-1 Recombinant rabbit monoclonal antibody (Invitrogen) at 1:1000 dilution overnight at 4°C. Then, the membranes were washed and incubated with rabbit-anti-peroxidase-conjugated goat IgG (Abcam Antibodies) at 1:1000 dilution and the signal was detected after incubation of Clarity Max^{™} Western ECL Substrate (BIO-RAD) using Versa Doc imaging System (BIO-RAD). The resulting bands were normalized on β-Tubulin (Abcam Antibodies) and analyzed using Quantity One software version 4.6.3.

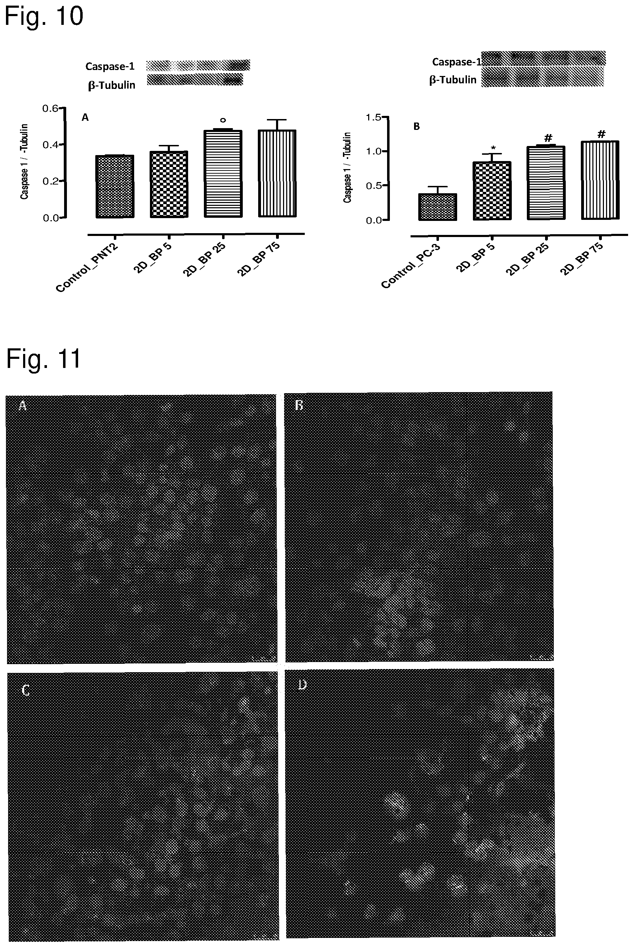

The results shown in Figure 10 demonstrated that 2D bP without NIR, at concentration of 5 µg/mL, did not induce caspase-1 expression in PNT-2 cells after 48 hours of exposure thus confirming the data observed on PNT-2 viability (Fig. 10A). Also the increase in a concentration dependent manner (from 5 to 75 µg/mL) of caspase-1 expression is in line with PNT-2 cell viability results (Fig. 10A). Conversely, 2D bP effect on PC-3 cells in caspase-1 induction is significant starting from 5 µg/mL (Fig. 10B). This result validated the cytotoxic effect of 2D bP in PC-3 related to an increase in ROS production.

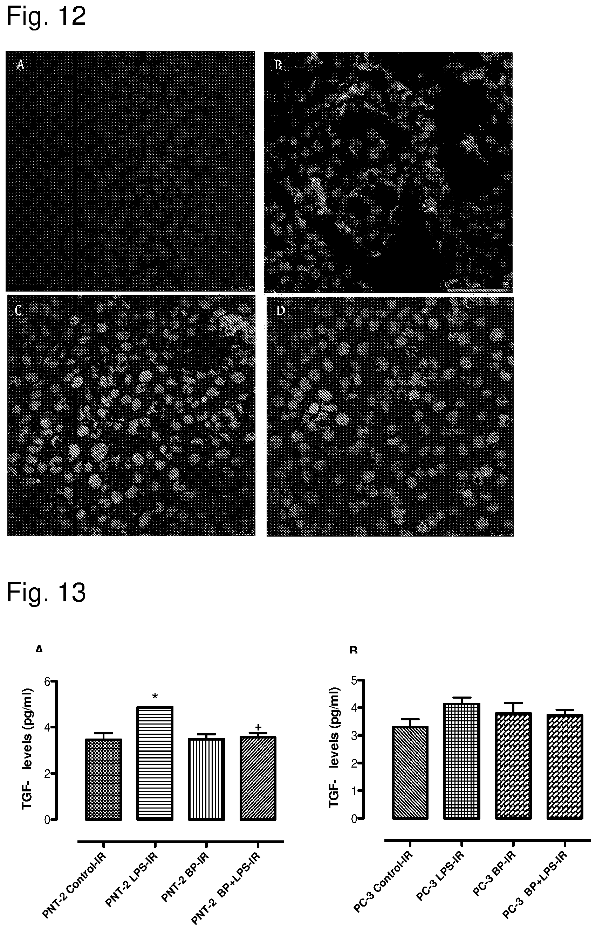

Western blot data were confirmed using immunofluorescence analysis. Cells were processed as previous describes for phalloidin detection and monoclonal rabbit-caspase-1 antibody (1:200 dilution) was incubated in each slide at 4°C overnight. After three washes with PBS, cells were treated for 2 hours with FITC conjugated secondary antibody (goat-antirabbit) at dilution of 1:500. Later, cells were washes thrice with PBS and incubated with DAPI (10 µg/mL). Finally, cells were washed other three times with PBS and observed at the confocal microscope. Figure 11 showed that 2D bP (5 µg/mL) did not induce caspase-1 expression in PNT-2 cells. Caspase-1 expression in PNT-2 cells is visible at higher concentrations of 2D bP (25 and 75 µg/mL). This result is in line with data obtained on cell viability. Conversely, as showed in Figure 12, 2D bP at concentration of 5 µg/mL was able to induce caspase-1 expression in PC-3 cells thus suggesting a selective apoptosis induction in cancer cells.

### Example 2.6.2 - TGF-β secretion

At cellular level, the up-regulation of TGF-β induces the pro-apoptotic function, conversely receptor inactivation causes malignant transformation. In order to understand a possible involvement of TGF-β signalling in the inhibition of PC-3 proliferation, TGF-β levels induced by LPS in PC-3 were compared to those measured in PNT-2.

To compare the behavior of healthy and cancer cells, PNT-2 and PC-3 treated with 2D bP without NIR-irradiation were exposed to LPS at concentration of 1µg/mL for 24 hours and TGF-β secretion was tested. TGF-β values were quantified as marker of oxidative stress related to cell apoptosis using commercial ELISA kits (Affimetrix Italia, SRL). The results shown in Figure 13 demonstrated that not irradiated 2D bP significantly decreased TGF-β levels induced by LPS in PNT-2 but not in PC-3 thus confirming a pro-apoptotic activity of 2D bP in cancer cells.

### Example 2.7 - Effect of 2D bP nanosheets on inflammatory response in PNT-2

To evaluate 2D bP anti-inflammatory potential on normal prostate tissue, the levels of inflammatory mediators (nitrites, IL-10, IL-6 and COX-2 levels) produced by cells after LPS (1µg/mL) stimulation for 3 days for mimicking the flogistic microenvironment were measured.

### Example 2.7.1 - IL10 and IL-6 levels

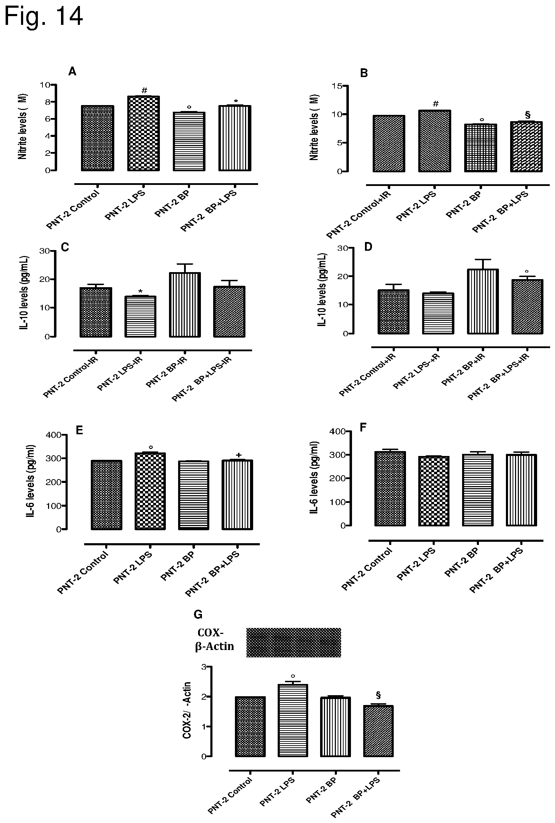

Pro- and anti- inflammatory interleukins secretion after 2D bP (5 µg/mL) treatment (with and without NIR-irradiation) was measured by using commercial ELISA kits (Affimetrix Italia, SRL) according to the manufacturer's instructions. After 24 hours of seeding (1×10⁵ cells /well), flogistic stimulus was obtained using LPS at concentration of 1µg/mL. After 3 days, interleukin-10 (IL-10) and interleukin-6 (IL-6) levels in PNT-2 supernatants were quantified. The results suggested that irradiated and not irradiated 2D bP stimulated the production of anti-inflammatory cytokines such as IL-10 (Fig. 14C,14D) and in parallel was able to significantly inhibit pro-inflammatory cytokines (IL-6) production but only without irradiation (Fig. 14E,14F).

### Example 2.7.2 - nitrites levels

Then, on the same supernatants nitrites levels were also analyzed using Griess assay to confirm LPS stimulation after 72 hours of exposure. LPS (1µg/mL) was able to induce high levels of nitric oxide in PNT-2 after 3 days of stimulation compared to control (Fig. 14A,14B). Irradiated and not irradiated 2D bP without LPS did not change basal levels of nitrites in PNT-2 cells (Fig. 14A,14B). By contrast, 2D bP significantly reduced nitric oxide production induced by LPS levels. These results are in agreement with the ability of 2D bP to inhibit the activation of pathway involved in oxidative stress (index of inflammation) in physiological conditions as previously discussed.

### Example 2.7.3 - COX-2 proteins expression

To study the anti-inflammatory potential of 2D bP on healthy prostate cells (PNT-2) stimulated with LPS at concentration of 1µg/mL for 72 hours, COX-2 [rabbit anti-COX-2 (Bioss Antibodies) at 1:200 dilution] expression was detected with western blot analysis as previous described for caspases-1. The resulting bands were normalized on β-actin (Abcam Antibodies) and analyzed using Quantity One software version 4.6.3. As showed in Figure 14G, 2D bP inhibited COX-2 expression in PNT-2 cells stimulated by LPS.

### Example 2.7.4 - SOD (superoxide dismutase) activity in PNT-2 culture

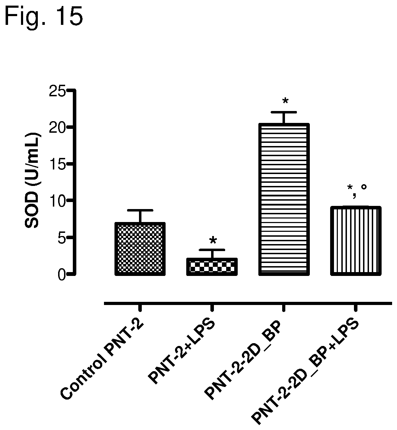

SOD is an enzyme involved in specific inhibition of superoxide anion free radical (O₂⁻) thus reducing the formation of nitrite and inflammatory cascade activation. Superoxide radical scavenging capacity of 2D bP at day 3 of LPS exposure in PNT-2 was analyzed to confirm 2D bP anti-inflammatory and antioxidant effect in normal prostate tissues. Superoxide dismutase (SOD) assay was performed using the Total SOD assay Kit Elabscience^{®} (Hydroxylamine method) according to manufacturer's instructions. Absorbance readings at 550 nm were recorded using a spectrophotometer (Victor X3 Multilabel Plate Reader, Perkin Elmer) and SOD activity was calculated as Inhibition ration=OD _{control}-OD ₛₐₘₚₗₑ/OD _{control} * 100% and expressed as SOD U/mL. Figure 15 showed LPS treatment for 3 days produced a significant decrease in SOD activity compared to control without flogistic stimulus, 2D bP exposure counteracted LPS-induced reduction in SOD activity. Moreover, 2D bP without LPS stimulation significantly induced basal SOD activity (Figure 15).

The present invention has been disclosed with particular reference to some specific embodiments thereof, but it should be understood that modifications and changes may be made by the persons skilled in the art without departing from the scope of the invention as defined in the appended claims.

## Claims

1. 2D exfoliated black phosphorus for use in the treatment of prostate cancer.

2. 2D exfoliated black phosphorus for use according to claim 1, wherein said prostate cancer is selected from the group consisting of primary prostate cancer and advanced prostate cancer.

3. 2D exfoliated black phosphorus for use according to claim 1, wherein said 2D exfoliated black phosphorus is used to treat organ confined prostate cancer cells.

4. 2D exfoliated black phosphorus for use according to claim 1 without phototherapy.

5. 2D exfoliated black phosphorus for use according to claim 1 with phototherapy.

6. 2D exfoliated black phosphorus for use according to claim 5, wherein said phototherapy is selected from the group consisting of photothermal therapy (PTT) or photodynamic therapy (PDT).

7. 2D exfoliated black phosphorus for use according to claim 5, wherein said phototherapy comprises the use of electromagnetic radiation selected from the group consisting of radiofrequency, microwaves, near infrared irradiation, or visible light.

8. 2D exfoliated black phosphorus for use according to any one of the preceding claims 1 to 7, wherein said 2D exfoliated black phosphorus is locally administered within the prostate tumor mass.

9. 2D exfoliated black phosphorus for use according to claim 8, wherein said 2D exfoliated black phosphorus is dispersed in a physiological injectable solution.

10. 2D exfoliated black phosphorus for use according to claim 9, wherein said 2D exfoliated black phosphorus is dispersed in said solution as such or incorporated into a carrier able to release the 2D exfoliated black phosphorus within the prostate tumor mass.

## Patentansprüche

1. 2D exfolierter schwarzer Phosphor zur Verwendung bei der Behandlung von Prostatakrebs.

2. 2D exfolierter schwarzer Phosphor zur Verwendung nach Anspruch 1, wobei der Prostatakrebs ausgewählt ist aus der Gruppe bestehend aus primärem Prostatakrebs und fortgeschrittenem Prostatakrebs.

3. 2D exfolierter schwarzer Phosphor zur Verwendung nach Anspruch 1, wobei der 2D exfolierte schwarze Phosphor verwendet wird, um organbegrenzte Prostatakrebszellen zu behandeln.

4. 2D exfolierter schwarzer Phosphor zur Verwendung nach Anspruch 1 ohne Phototherapie.

5. 2D exfolierter schwarzer Phosphor zur Verwendung nach Anspruch 1 mit Phototherapie.

6. 2D exfolierter schwarzer Phosphor zur Verwendung nach Anspruch 5, wobei die Phototherapie ausgewählt ist aus der Gruppe bestehend aus photothermischer Therapie (PTT) oder photodynamischer Therapie (PDT).

7. 2D exfolierter schwarzer Phosphor zur Verwendung nach Anspruch 5, wobei die Phototherapie die Verwendung von elektromagnetischer Strahlung umfasst, die ausgewählt ist aus der Gruppe bestehend aus Radiofrequenz, Mikrowellen, Nahinfrarotstrahlung oder sichtbarem Licht.

8. 2D exfolierter schwarzer Phosphor zur Verwendung nach einem der vorhergehenden Ansprüche 1 bis 7, wobei der 2D exfolierte schwarze Phosphor lokal innerhalb der Prostatatumormasse verabreicht wird.

9. 2D exfolierter schwarzer Phosphor zur Verwendung nach Anspruch 8, wobei der 2D exfolierte schwarze Phosphor in einer physiologischen injizierbaren Lösung dispergiert ist.

10. 2D exfolierter schwarzer Phosphor zur Verwendung nach Anspruch 9, wobei der 2D exfolierte schwarze Phosphor in der Lösung als solche dispergiert ist oder in einem Träger inkorporiert ist, der den 2D exfolierten schwarzen Phosphor in der Prostatatumormasse freisetzen kann.

## Revendications

1. Phosphore noir exfolié 2D pour l'utilisation dans le traitement du cancer de la prostate.

2. Phosphore noir exfolié 2D pour l'utilisation selon la revendication 1, ledit cancer de la prostate étant sélectionné dans le groupe constitué du cancer primaire de la prostate et du cancer avancé de la prostate.

3. Phosphore noir exfolié 2D pour l'utilisation selon la revendication 1, ledit phosphore noir exfolié 2D étant utilisé pour traiter des cellules cancéreuses de prostate confinées dans un organe.

4. Phosphore noir exfolié 2D pour l'utilisation selon la revendication 1, sans photothérapie.

5. Phosphore noir exfolié 2D pour l'utilisation selon la revendication 1, avec photothérapie.

6. Phosphore noir exfolié 2D pour l'utilisation selon la revendication 5, ladite photothérapie étant sélectionnée dans le groupe constitué de la thérapie photothermique (PTT) ou de la thérapie photodynamique (PDT).

7. Phosphore noir exfolié 2D pour l'utilisation selon la revendication 5, ladite photothérapie comprenant l'utilisation d'un rayonnement électromagnétique sélectionné dans le groupe constitué d'une radiofréquence, des micro-ondes, de l'irradiation dans le proche infrarouge, ou de la lumière visible.

8. Phosphore noir exfolié 2D pour l'utilisation selon l'une quelconque des revendications précédentes de 1 à 7, ledit phosphore noir exfolié 2D étant localement administré à l'intérieur de la masse tumorale prostatique.

9. Phosphore noir exfolié 2D pour l'utilisation selon la revendication 8, ledit phosphore noir exfolié 2D étant dispersé dans une solution physiologique injectable.

10. Phosphore noir exfolié 2D pour l'utilisation selon la revendication 9, ledit phosphore noir exfolié 2D étant dispersé dans ladite solution tel quel ou incorporé dans un support capable de libérer le phosphore noir exfolié 2D à l'intérieur de la masse tumorale prostatique.
